Europäisches Patentamt

European Patent Office

Office européen des brevets

(11) Numéro de publication: **0 358 579**
**A2**

## (12) DEMANDE DE BREVET EUROPEEN

(21) Numéro de dépôt: **89420270.4**

(22) Date de dépôt: **25.07.89**

(51) Int. Cl.⁵: **A 61 B 17/58**
C 07 D 235/08,
C 07 D 235/10,
C 07 D 405/06, C 07 F 7/18,
A 61 K 31/415

(30) Priorité: **28.07.88 FR 8810530**

(43) Date de publication de la demande:
**14.03.90 Bulletin 90/11**

(84) Etats contractants désignés:
**AT BE CH DE ES GB GR IT LI LU NL SE**

(71) Demandeur: **ICP FRANCE, Société Anonyme**
**Zac Val Varinot B.P. No. 91 Boulevard Maréchal Juin**
**F-52003 Chaumont Cédex (FR)**

(72) Inventeur: **Michel, Jean Pierre**
**Rochetaillée**
**F-52210 Arc en Barrois (FR)**

**Landanger, Joel**
**Le Moulin de la Renne La Villeneuve au Roi**
**F-52230 Colombey les Deux Eglises (FR)**

(74) Mandataire: **Dupuis, François**
**Cabinet Charras 3 Place de l'Hôtel-de-Ville BP 203**
**F-42005 St. Etienne Cédex 1 (FR)**

(54) **Dispositif de visée pour le positionnement d'au moins un organe de fixation à travers un implant du type clou centromédullaire.**

(57) L'invention se rattache au secteur technique des sciences médicales.

Le dispositif selon l'invention comprend un organe (1) du type viseur monté dans un support (5) avec capacité de déplacement et de réglage en longueur, en rotation, en flexion et en translation, en étant apte à reconnaître l'obliquité du rayon X en le matérialisant afin de positionner très exactement l'organe du type viseur (1), au droit et en alignement de la partie du clou devant recevoir l'organe de fixation, ledit organe viseur (1) étant susceptible d'être remplacé par un élément de guidage apte à assurer la mise en place dudit organe de fixation.

FIG.5

EP 0 358 579 A2

Description

## Dispositif de visée pour le positionnement d'au moins un organe de fixation à travers un implant, du type clou centro-médullaire.

L'invention se rattache au secteur technique des sciences médicales.

On sait que les clous, en permettant aux forces représentées par le poids du corps et son énergie cinétique de s'exercer au niveau du foyer des fractures, favorisent la consolidation. De plus en plus, la plupart des clous sont verrouillés au moyen de vis ou autres organes d'assemblage, afin d'éviter les rotations, de restaurer en toute sécurité les longueurs et de pouvoir traiter un certain nombre de fractures épiphysaires.

Cependant, la technique de verrouillage s'avère très délicate, et nécessite un appareil connu sous le nom "amplificateur de brillance" qui génère des rayons X afin de pouvoir visualiser par exemple le ou les trous que présentent les clous pour le passage des broches ou vis de fixation.

Un problème important résulte de la déformation du clou lors de son introduction, tant en torsion qu'en flexion. De plus, le clou, dans sa partie distale, ne suit pas toujours la ligne droite ou fait dévier l'extrémité osseuse distale, perturbant ainsi les rapports et les repères éventuels entre ledit clou et l'os.

Selon un premier procédé de verrouillge connu, on tente d'aligner deux trous au moyen de l'amplificateur de brillance, à savoir un trou que présente le clou et un trou que présente un viseur fixé à l'amplificateur. Pour avoir un résultat correct, il est nécessaire que l'image du trou soit ronde. Or, compte tenu des déformations possibles susindiquées, très souvent le trou est ellipsoïdal.

Il apparaît également des problèmes d'instabilité de l'ensemble de l'amplificateur et du viseur, non solidaire du clou. De telles difficultés de manipulation pour placer le viseur dans l'axe du trou du clou, nécessitent une personne particulièrement qualifiée.

Selon un autre mode opératoire connu, on utilise un viseur tenu à la main en ayant pour objectif, toujours en combinaison avec un générateur de rayons X, d'aligner les deux trous, à savoir celui du viseur et celui du clou. Pour qu'un tel mode soit efficace, il faudrait que le practicien puisse, à la fois, rectifier et intégrer les différentes positions de l'espace au fur et à mesure des mouvements imprimés au viseur de manière à obtenir la concentricité des trous du viseur et des clous et la sphéricité de ces deux tours ; la bonne position étant trouvée, il convient encore de la maintenir.

Une telle façon de procéder peut durer jusqu'à une heure, avec l'irradiation correspondante en rayons X. On conçoit que cela ne peut être acceptable.

Pour tenter de remédier à ces inconvénients, on a proposé un dispositif de visée comportant un organe support sur lequel est monté l'organe viseur en tant que tel. Cet état de la technique peut-être illustré par le brevet US n° 4.667.664.

Il apparait cependant que le résultat obtenu n'est pas satisfaisant. Des problèmes importants de réglage apparaissent compte-tenu des moyens de mises en oeuvre, de sorte que la façon de procéder pour tenter d'obtenir les différents réglages, notamment au niveau de l'alignement du viseur et du ou des trous de l'implant, ne relève pas d'une démarche et d'une déduction logiques.

L'invention s'est fixée pour but de remédier à ces inconvénients.

Le problème posé est donc de pouvoir assurer le verrouillage d'un clou d'une manière simple et rapide, en évitant le maximum de contact avec les rayons X.

Le dispositif de visée, selon l'invention, est conformé pour s'adapter à tout type de clou et comprend un organe du type viseur monté dans un support avec capacité de déplacement et de réglage en longueur, en rotation, en flexion et en translation, en étant apte à reconnaître l'obliquité du rayon X en le matérialisant afin de positionner très exactement l'organe du type viseur, au droit et en alignement de la partie du clou devant recevoir l'organe de fixation.

Pour résoudre le problème posé de tenir compte d'une éventuelle déformation de l'image du trou du clou l'organe viseur est conformé pour réduire par projection plane les trois dimensions de l'espace à une seule en étant apte à fonctionner correctement même si la partie de l'implant devant recevoir l'organe de fixation, n'apparaît pas rigoureusement ronde.

Avantageusement, l'organe viseur est un cylindre radio-transparent aux rayons X à l'intérieur duquel sont positionnés des éléments notamment en acier radio-opaque aptes à délimiter, en combinaison, une mire. Les éléments en acier radio-opaque, sont constitués par quatre lames rectangulaires de faible épaisseur et disposées en croix en étant décalées angulairement de 90°, lesdites lames affleurant la périphérie et les parties d'extrémité du cylindre tout en délimitant une partie centrale libre.

Pour résoudre le problème des déformations des clous après leur mise en place, qui perdent ainsi leurs rapports intrinsèques initiaux, la partie du support recevant l'organe viseur est constituée par un ensemble mobile monté avec capacité de déplacement en translation et de pivotement angulaire dans un plan horizontal en étant assujetti à des moyens de blocage en position, ledit ensemble mobile recevant l'organe viseur avec capacité d'orientation angulaire et de blocage en position.

Pour résoudre le problème posé de l'instabilité du viseur, le support présente une partie faisant office de rail de guidage coopérant avec l'élément de fixation avec capacité de coulissement et de blocage en translation , ladite partie étant montée avec capacité d'orientation angulaire par rapport à une autre partie du support recevant l'organe viseur, tandis que l'élément de fixation est conformé pour coopérer avec une partie de l'implant ou de l'os.

L'invention est exposée ci-après plus en détail à l'aide des dessins annexés dans lesquels :

Les figures 1 à 3 montrent le principe de visée selon l'invention.

La figure 4 est une vue en perspective d'une forme de réalisation du viseur.

La figure 5 est une vue de face du dispositif.

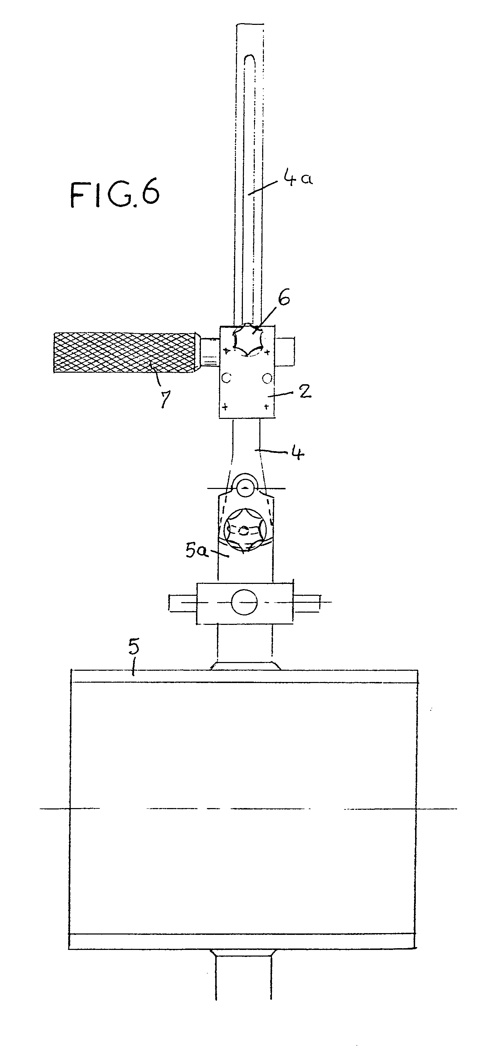

La figure 6 est une vue en plan correspondant à la figure 5.

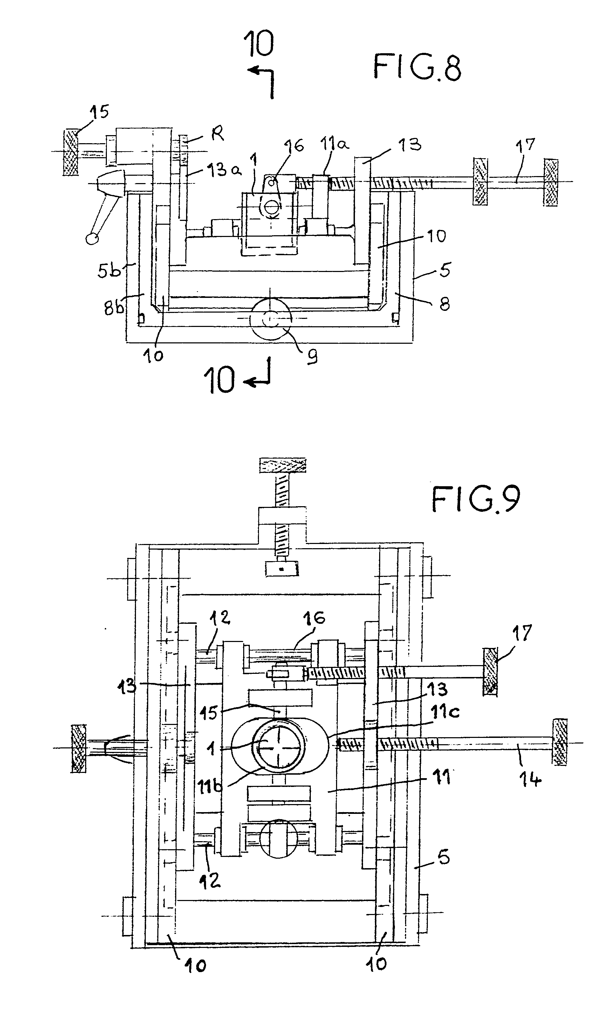

La figure 7 est une vue en coupe transversale considérée selon la ligne 7-7 de la figure 5.

La figure 8 est une vue de face de la partie du support recevant l'organe viseur.

La figure 9 est une vue en plan correspondant à la figure 8.

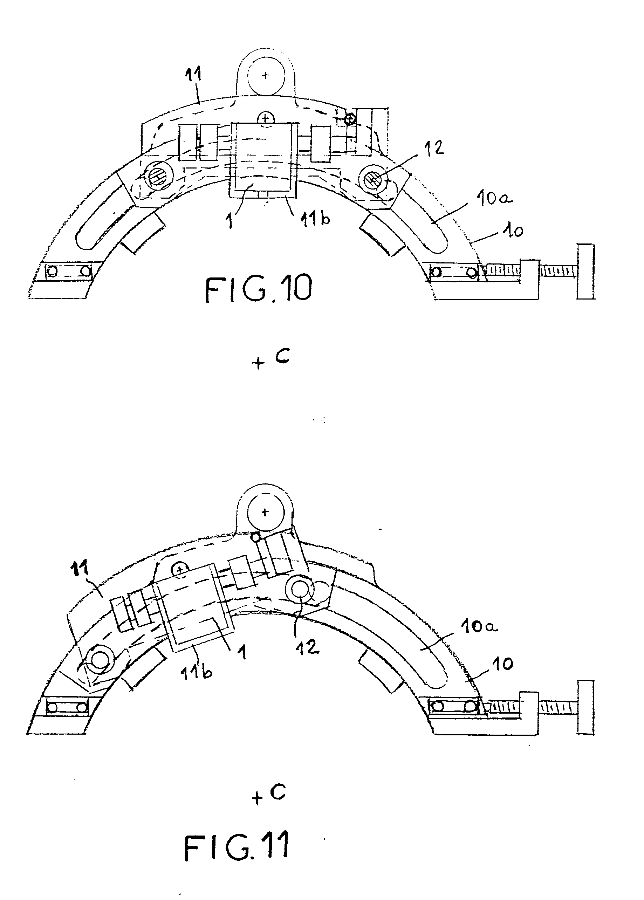

La figure 10 est une vue en coupe transversale considérée selon la ligne 10-10 de la figure 8.

La figure 11 est une vue semblable à la figure 10 montrant une autre position de l'ensemble mobile.

Le dispositif selon l'invention comprend un organe du type viseur (1) monté dans un support (5), avec capacité de déplacement et d'orientation des différentes directions en étant apte à reconnaître l'obliquité du rayon X en le matérialisant.

Le viseur en tant que tel est constitué par un cylindre radio-transparent, cercle de métal radio-opaque ou autre, apte à réduire par projection plane les trois dimensions de l'espace à une seule. Le viseur est en outre conformé pour être efficace même si l'image du trou (C1) du clou (C) a une forme d'ellipse, ce qui facilite grandement le réglage de l'amplificateur de brillance.

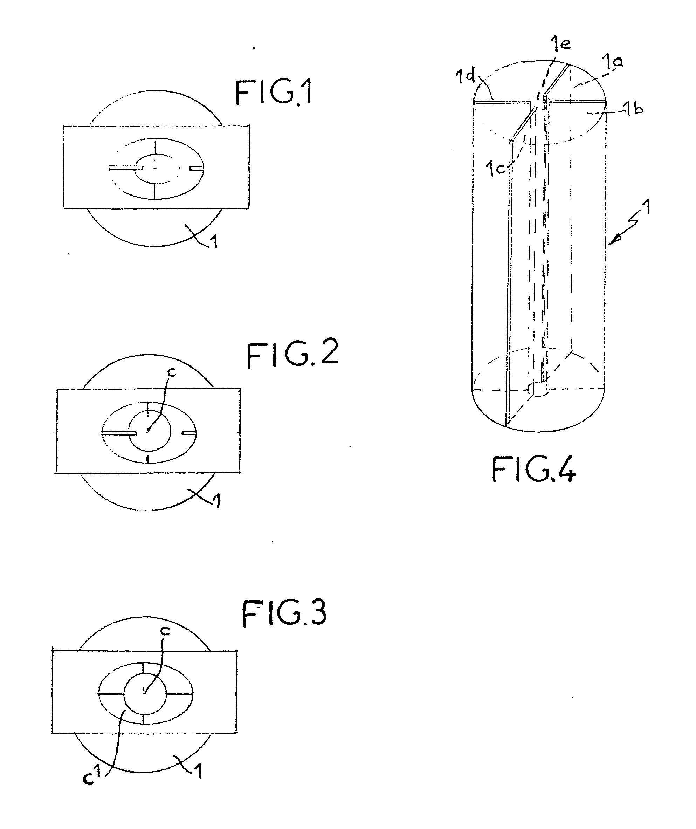

Dans l'exemple illustré (figure 4), le cylindre de visée (1) est transparent aux rayons X et est équipé intérieurement de quatre lames verticales (1a), (1b), (1c) et (1d) en acier radio-opaque de faible épaisseur. Ces lames sont positionnées dans des plans orthogonaux, en étant décalées angulairement de 90° et en affleurant la périphérie et les parties d'extrémité du cylindre (1) tout en délimitant une partie centrale libre (1e). Cette partie centrale a des dimensions variables en fonction du trou à viser et de l'élément de fixation à positionner.

Bien évidemment, ce cylindre de visée peut avoir d'autres configurations telles que par exemple deux lames rectangulaires sur un même diamètre ou sur deux diamètres différents.

Comme indiqué, le cylindre de visée permet de déterminer l'obliquité de rayons X en provenance du générateur (G) et fait office de viseur en même temps. Si le rayon X est parfaitement à la verticale du cylindre (1), la matérialisation dudit rayon est une croix dont le centre (c) est un cercle correspondant à l'image de la partie centrale libre (1e) dudit cylindre. Dès que le rayon X s'écarte de la verticale, trois phénomènes visibles apparaissent, d'une manière isolée, ou en combinaison :

- épaississement de l'une ou de plusieurs branches de la croix,
- ovalisation de l'image de la partie centrale libre (1e),
- empiètement de l'une des branches de la croix sur l'image de la partie centrale libre.

Ce cylindre de visée, détecteur d'obliquité de rayons X possède donc, de par ses caractéristiques, la possibilité de se placer parfaitement au centre du rayon principal.

Il permet aussi, grâce à l'analyse de forme informatique de donner en degré l'obliquité du rayon.

Il permettra donc une visée informatique automatique par mise en place correcte du viseur quelle que soit la position du rayon X incident.

Comme indiqué, une fois placé parfaitement perpendiculairement au rayon principal, le viseur apparaît sous forme d'une fine croix avec un rond blanc en son centre qui correspond "à la ligne de tir". Etant donné que ce rond a une certaine surface, variable selon le viseur utilisé, et correspondant à la section de l'élément de fixation à utiliser, celui-ci est situé à l'intérieur de l'image visée, l'élément de fixation y passera très précisément quelle que soit la forme de ladite image. Comme indiqué dans la suite de la description, il suffit ensuite de changer ce cylindre de visée et de le remplacer par un organe apte à diriger un quelconque outil tranchant de perçage ou de fixation.

Il convient donc d'amener le cylindre de visée (1) au niveau de la cible constituée par le clou (C1) du clou (C) et de positionner ledit cylindre au droit du rayon X principal, et de tenir compte des déformations du clou. Il en résulte un agencement particulier du support (5).

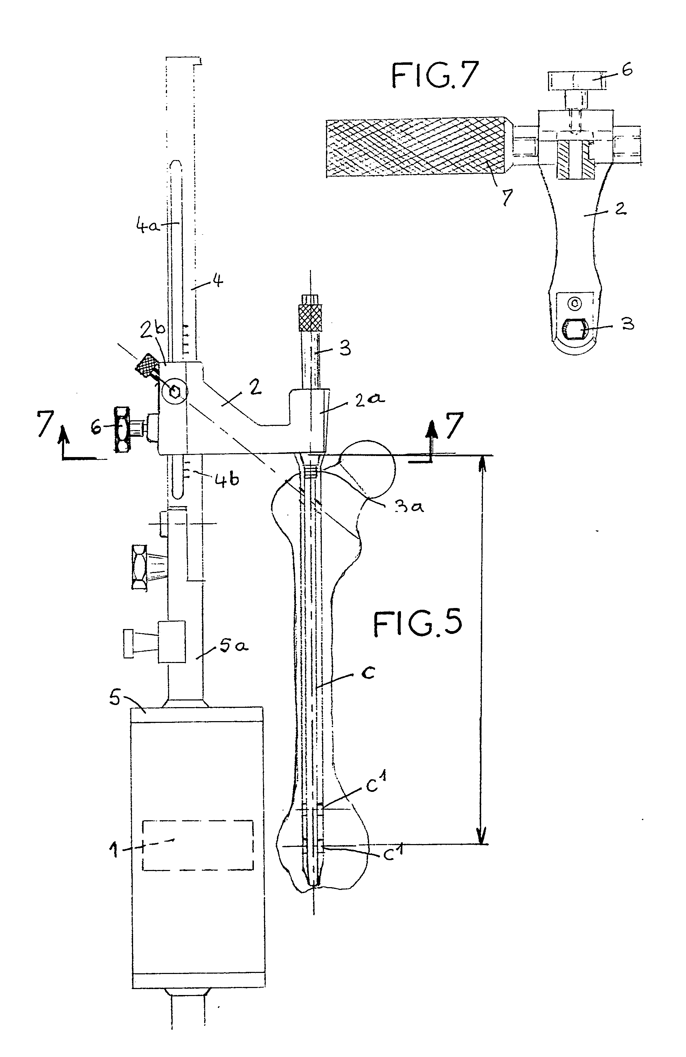

Comme le montre la figure 5, le support (5) coopère avec un élément de fixation (2) conformé pour être rendu solidaire d'une partie du clou (C) ou de l'os. Dans l'exemple illustré, l'embase (2a) de l'élément de fixation (2) est agencée pour être accouplée, d'une manière démontable, à l'extrémité du clou (C) débordant de l'épiphyse de l'os. L'embase (2a) reçoit une tige de manoeuvre (3) présentant un nez fileté (3a) apte à être vissé dans la partie débordante du clou, en respectant, dans ces conditions, les caractéristiques proximales de chaque clou.

La partie supérieure de l'élément (2) présente un tunnel de guidage (2b) recevant à libre coulissement un rail (4) accouplé d'une manière démontable, avec capacité d'orientation angulaire, en bout d'une partie (5a) que présente un berceau (5) du support. Une lumière oblongue (4a) établie dans la partie médiane du rail (4) coopère avec une vis de réglage (6) de l'élément (2), afin d'assurer son blocage en translation. En outre des repères (4b) sont formés sur le côté du rail (4) pour permettre de régler la longueur de l'entraxe (L) entre l'organe viseur (1) et l'élément de fixation (2) qui doit correspondre à la longueur entre l'extrémité du clou et le trou de verrouillage (C1) dudit clou.

Une poignée de préhension (7) est solidaire transversalement de l'élément de fixation (2).

La partie du support (5) recevant l'organe viseur (1) est constitué par un ensemble mobile monté avec capacité de déplacement en translation et de pivotement angulaire dans un plan horizontal, en étant assujetti à des moyens de blocage en position. Cet ensemble mobile comprend un berceau mobile (8) monté transversalement à coulissement guidé à l'intérieur du berceau fixe (5), en étant assujetti à un moyen de réglage et de blocage en position (9). Les flasques (5b) et (8b) respectivement des berceaux (5) et (8), sont profilés en arc de cercle.

En appui sur la partie interne des flasques (8b), sont montés deux éléments fixes profilés en arc de cercle (10) présentant chacun une lumière (10a) semi-circulaire dont le centre correspond à l'axe du clou.

Les éléments (10) reçoivent deux à deux un chariot (11) porté par deux tiges de guidage parallèle (12) coopérant avec les lumières (10a) desdits éléments. Ces tiges de guidage (12) sont entretoisées à leur extrémité par des flasques profilés (13) dont l'un est agencé pour permettre l'entraînement circulaire de l'ensemble du chariot (11), en combinaison avec les lumières (10a) des éléments (10).

Par exemple, l'un des flasques (13) peut présenter une denture (13a) coopérant avec une roue dentée (R) assujettie à un organe de manoeuvre (20) porté par l'élément (10) correspondant. En outre, le chariot (11) est monté sur les tiges (12) avec capacité de déplacement et de réglage en translation au moyen par exemple d'une vis de manoeuvre (14) portée par l'autre flasque (13) et vissée dans un bossage (11a) du chariot.

Le chariot présente très sensiblement dans sa partie centrale, un fût (11b) apte à recevoir l'organe viseur (1). Ce fût (11b) est supporté par deux axes tourillons (15) pour pivoter angulairement dans une lumière (11c) du chariot, dans un plan perpendiculaire à celui du déplacement angulaire dudit chariot (11). Les axes tourillons (15) sont assujettis à un système d'accouplement (16) manoeuvré par une vis (17) engagée dans une partie correspondante du chariot (11).

Dans ces conditions, il apparaît que l'organe viseur (1) peut être déplacé et orienté dans tous les plans de l'espace, compte tenu de la conception particulière du support telle que décrite. Lorsque la visée est terminée, il suffit de remplacer, après blocage de l'ensemble mobile, l'organe viseur (1), par un élément de tout type connu et approprié et apte à permettre le guidage d'un outil du type broche.

A noter que le berceau fixe (5) du support, reçoit, d'une manière démontable, un système de stabilisation coopérant avec l'os, pour supprimer le porte-à-faux. Ce système est constitué par une tige horizontale (18) solidaire du berceau (5) et recevant avec capacité de réglage, en position, une tige d'appui verticale (19).

Dans la forme de réalisation décrite et illustrée, les différents réglages s'opèrent au moyen de molettes manuelles, sans pour cela exclure d'autres systèmes. Par exemple, ces molettes peuvent être remplacées par des douilles mâles à quatre pans aptes à coopérer avec des douilles femelles complémentaires et accouplées à un organe d'entraînement du type perceuse. Il en résulte un réglage à distance évitant ainsi toute irradiation par les rayons X par le praticien.

On prévoit également d'asservir l'ensemble du dispositif à un programme informatique, les molettes de réglage étant remplacées par des moteurs pas à pas.

L'invention trouve une application particulièrement avantageuse pour le verrouillage des clous centro-médullaires, et plus généralement pour permettre une visée précise à travers un os.

**Revendications**

-1- Dispositif de visée pour le positionnement précis et rapide, en combinaison avec un générateur de rayons X, d'au moins un organe de fixation à travers un implant notamment du type clou, placé dans le canal médullaire d'un os, comprenant un organe (1) du type viseur monté dans un support (5) avec capacité de déplacement et de réglage, en étant apte à reconnaître l'obliquité du rayon X en le matérialisant afin de positionner l'organe du type viseur (1), au droit et en alignement de la partie du clou devant recevoir l'organe de fixation, caractérisé en ce que la partie (5) du support recevant l'organe viseur (1) est constituée par un ensemble mobile monté avec capacité de déplacement en translation et de pivotement angulaire dans un plan horizontal en étant assujetti à des moyens de blocage en position, ledit ensemble mobile recevant l'organe viseur avec capacité d'orientation angulaire et de blocage en position.

-2- Dispositif selon la revendication 1, caractérisé en ce que l'ensemble mobile comprend un berceau (8) monté transversalement à coulissement guidé à l'intérieur de la partie fixe (5) du support en étant assujetti à un moyen de réglage et de blocage en position, les flasques dudit berceau (8) recevant deux éléments fixes profilés en arc de cercle (10) et présentant chacun une lumière (10a) semi-circulaire dont le centre correspond à l'axe du clou.

-3- Dispositif selon la revendication 2, caractérisé en ce que les éléments (10) reçoivent deux à deux un chariot (11) porté par deux tiges de guidage parallèles (12) coopérant avec les lumières (10a) desdits éléments, lesdits tiges (12) étant entretoisées à leur extrémité par des flasques profilés (13) dont l'un est agencé pour permettre l'entraînement circulaire de l'ensemble du chariot (11) en combinaison avec lesdites lumières (10a).

-4- Dispositif selon la revendication 3, caractérisé en ce que le chariot (11) est monté sur les tiges (12) avec capacité de déplacement et de réglage en translation au moyen d'un organe (14) porté par l'autre flasque (13) et coopérant avec une partie dudit chariot (11).

-5- Dispositif selon les revendications 3 et 4, caractérisé en ce que le chariot (11) présentent très sensiblement dans sa partie médiane un fût (11b) apte à recevoir l'organe viseur (1), ledit fût étant supporté par deux axes tourillons (15) pour pivoter angulairement dans une lumière (11c) du chariot et dans un plan perpendiculaire à celui du déplacement circulaire du chariot, lesdits axes tourillons étant assujettis à un système d'accouplement coopérant avec un organe de manoeuvre et de réglage (17).

-6- Dispositif selon la revendication 1, caractérisé en ce que l'organe viseur (1) est un cylindre radio-transparent aux rayons X à l'intérieur duquel sont positionnés des éléments notamment en acier radio-opaque, constitués par quatre lames rectangulaires de faible épaisseur et disposées en croix en étant décalées angulairement de 90 °, lesdites lames affleurant la périphérie et les parties d'extrémité du cylindre tout en délimitant une partie central libre (1e).

-7- Dispositif selon la revendication 1, caractérisé en ce que le support présente une partie faisant office de rail de guidage coopérant avec un élément de fixation (2) avec capacité de coulissement et de blocage en translation, ladite partie (4) étant montée avec capacité d'orientation angulaire par rapport à une autre partie (5a) du support recevant l'organe viseur, tandis que l'élément (2) est conformé pour coopérer avec une partie de l'implant ou de l'os.

-8- Dispositif selon la revendication 7, caractérisé en ce que l'embase (2a) de l'élément de fixation (2) est agencée pour être accouplée, d'une manière démontable, à l'extrémité du clou débordant notamment de l'épiphyse de l'os.

-9- Dispositif selon la revendication 7, caractérisé en ce que des repères (4b) sont formés sur le côté de la partie du support faisant office de rail (4) pour permettre de régler la longueur de l'entraxe entre l'organe viseur et l'élément de fixation qui doit correspondre à la longueur entre l'extrémité du clou et la partie de verrouillage dudit clou.

-10- Dispositif selon l'une quelconque des revendications 5 à 13, caractérisé en ce que la partie (5) du support reçoit, de manière démontable, un système de stabilisation (18 - 19) apte à coopérer avec l'os.

FIG.1

FIG.2

c

FIG.3

c

c¹

1e
1d
1a
1b
1c
1

FIG.4

EP 0 358 579 A2

FIG.7

FIG.5

FIG.6

FIG.8

FIG.9

FIG.10

+ C

+ C

FIG.11